# EUROPEAN PATENT APPLICATION

(11) **EP 4 628 590 A1**
(43) Date of publication of application: **08.10.2025**
(21) Application number: 24832411.3
(22) Date of filing: 26.06.2024
(51) Int. Cl.: C12N 15/77, C07K 14/245, C12P 13/06

(54) **MICROORGANISM EXPRESSING THREONINE EXPORT PROTEIN RHTC, AND USE THEREOF FOR PRODUCING L-ISOLEUCINE**

(30) Priority: 28.06.2023 KR 20230083501
(71) Applicant: CJ CheilJedang Corporation, Seoul 04560 (KR)
(72) Inventor: KIM, Kyungrim, Seoul 04560 (KR); KIM, Heeyeong, Seoul 04560 (KR)
(74) Representative: Meissner Bolte Partnerschaft mbB
(86) International application number: PCT/KR2024/008841
(87) International publication number: WO 2025/005633

(57) **Abstract**

The present disclosure provides: an L-isoleucine-producing microorganism expressing threonine export protein RhtC; a method for producing L-isoleucine, comprising a step of culturing the microorganism; a method for increasing L-isoleucine productivity of the microorganism, comprising a step of introducing a threonine export protein RhtC gene into a cell; and a use thereof for producing L-isoleucine.

## Description

### [TECHNICAL FIELD]

### Cross-reference to related application(s)

The present disclosure claims the benefit of the priority based on Korean Patent Application No. 10-2023-0083501 filed on June 28, 2023, and the entire contents disclosed in the document of the corresponding Korean patent application are incorporated as a part of the present disclosure.

Throughout the present disclosure, numerous papers and patent documents are referenced and citations thereof are indicated. The disclosure contents of the cited papers and patent documents are incorporated by reference into the present disclosure in their entirety to more clearly describe the level of the technical field to which the present invention pertains and the contents of the present invention.

The present disclosure relates to a microorganism expressing threonine export protein RhtC and a use for producing L-isoleucine thereof, and relates to a microorganism expressing threonine export protein RhtC, a method for producing L-isoleucine using the microorganism, a method for increasing an L-isoleucine production capacity of the microorganism and/or a composition for producing L-isoleucine.

### [BACKGROUND ART]

L-isoleucine (Ile, I) is an essential amino acid, and is used in preparation of various products such as animal feed, food additives, medicines and the like. Since L-isoleucine has functions such as energy generation, hemoglobin production, blood sugar control, muscle generation, and repair and the like after metabolism, its use is increasing not only in fluids, nutrients, and sports nutrients, but also in the animal feed field.

Based on this trend, various attempts are being made to improve the production capacity in the method for production of L-isoleucine using various microorganisms including *Corynebacterium* sp. microorganisms.

Despite these efforts, development of technology to improve the production capacity of L-isoleucine is still required.

### [PRIOR ART]

(Patent document 1) US 10113190 B2 (2018.10.30)
(Patent document 2) US 2023-0012923 A1 (2023.01.19)

### [DISCLOSURE]

### [TECHNICAL PROBLEM]

The present disclosure provides a microorganism producing L-isoleucine expressing threonine export protein RhtC.

The present disclosure provides a composition for producing L-isoleucine comprising at least one selected from the group consisting of the threonine export protein RhtC, a polynucleotide encoding thereof, a vector comprising the polynucleotide, and a microorganism expressing threonine export protein RhtC.

The present disclosure provides a method for producing L-isoleucine, comprising culturing a microorganism expressing the threonine export protein RhtC.

The present disclosure provides a method for increasing an L-isoleucine production capacity of a microorganism, comprising introducing a polynucleotide encoding the threonine export protein RhtC into a microorganism.

The present disclosure provides a use for producing L-isoleucine, preparing a microorganism producing L-isoleucine, and/or increasing an L-isoleucine production capacity of a microorganism, of at least one selected from the group consisting of the threonine export protein RhtC, a polynucleotide encoding thereof, a vector comprising the polynucleotide, and a microorganism expressing the threonine export protein RhtC.

### [TECHNICAL SOLUTION]

The present disclosure develops a microorganism having an L-isoleucine production capacity or having an increased L-isoleucine production capacity by searching protein having a function of giving and/or increasing an L-isoleucine production capacity of a microorganism, and introducing it into a suitable host cell, thereby providing a microorganism comprising the protein, a use for producing L-isoleucine thereof, and related technologies.

### Definition of terms

In the present disclosure, the term "corresponding to" may refer to, for any polypeptide or polynucleotide, an amino acid sequence or nucleic acid sequence of the corresponding region to be identified by aligning the amino acid sequence or nucleic acid sequence of the polypeptide or polynucleotide with a specific amino acid sequence or nucleic acid sequence provided in the present disclosure. This sequence alignment may be done by a conventional alignment method, and for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277) and the like may be used, but not limited thereto, a sequence alignment program known in the art, pairwise sequence comparison algorithms and the like may be appropriately used.

In the present disclosure, that a polynucleotide (may be interchangeably used with "nucleic acid molecule" or "gene") or peptide (may be interchangeably used with "protein") "has or comprises a specific nucleic acid sequence (base sequence) or amino acid sequence or consists of the sequence" may mean that the polynucleotide or polypeptide essentially comprises the specific nucleic acid sequence (base sequence) or amino acid sequence, and it may be interpreted as comprising "a substantially equivalent sequence" (or not excluding the mutation) in which a mutation (deletion, substitution, modification, and/or addition) is applied to the specific nucleic acid sequence (base sequence) or amino acid sequence within a range of maintaining the original function and/or desired function of the polynucleotide or polypeptide.

In one embodiment, the nucleic acid sequence or amino acid sequence provided in the present disclosure may comprise one modified by common mutagenesis, for example, direct evolution and/or site-directed mutagenesis and the like within a range of maintaining the original functions or desired functions thereof.

In one embodiment, that a polynucleotide or polypeptide "comprises a specific nucleic acid sequence (base sequence) or amino acid sequence or consists of the sequence" may mean that the polynucleotide or polypeptide (i) essentially comprises the specific nucleic acid sequence (base sequence) or amino acid sequence, or (ii) consists of a nucleic acid sequence or amino acid sequence having homology or identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more (the upper limit may be 100% or less than 100%) with the specific nucleic acid (base sequence) or amino acid sequence, or essentially comprises it and maintains the original function and/or desired function. In one embodiment, the original function may be a threonine export function, and the desired function may mean an L-isoleucine production capacity (for example, a function of giving and/or increasing an L-isoleucine production capacity compared to a parent strain (unintroduced microorganism), when introduced into a host cell (microorganism)).

In the present disclosure, the term 'homology' or 'identity' refers to the degree to which two given amino acid sequences or base sequences are related, and may be expressed as a percentage. The terms homology and identity may be often used interchangeably.

In addition, as long as it is an amino acid sequence having this homology or identity and exhibiting functions corresponding to the threonine export protein (the original functions and/or desired functions), proteins having an amino acid sequence in which some sequences are deleted, modified, substituted or added may be comprised in the range of proteins subject to mutation of the present disclosure.

In the present disclosure, the term, "variant" refers to a protein or a gene encoding it in which at least one amino acid is different from a sequence defined by a certain sequence number for conservative substitution and/or modification, but the functions or properties of the protein are maintained. The variant may have various sequences by substitution, deletion or addition of one or more (for example, 1, 2, 3, etc.) amino acids. Such a variant may be generally identified by modifying at least one amino acid among the amino acid sequence of the protein, and evaluating properties of the modified protein. The function of the variant may be maintained at the same level, increased, or reduced to an insignificant extent, compared to the native protein. In addition, some variants may include variants in which one or more parts such as an N-terminal leader sequence or a transmembrane domain are removed. Other variants may include variants in which a part is removed from the N-terminus and/or C-terminus of mature protein. The term "variant" may be represented also by a mutant, modified, mutated protein/polynucleotide, mutant polypeptide/polynucleotide and the like, and it is not limited thereto as long as it is a term used in a mutated meaning.

For the purpose of the present disclosure, the variant may have maintained or increased activity (for example, original activity and/or desired activity) compared to wild-type or non-modified protein, but is not limited thereto.

The variant of the protein in the present disclosure may be produced by conservative substitution. The term "conservative substitution" refers to substituting one amino acid with another amino acid having similar structural and/or chemical properties. The variant may have for example, at least one conservative substitution, while still retaining one or more biological activities. This amino acid substitution may generally occur based on similarity in polarity, charge, solubility, hydrophobicity, hydrophilicity and/or amphipathic nature of residues. For example, among amino acids with an electrically charged side chain (electrically charged amino acid), positively charged (basic) amino acids may include arginine, lysine and histidine, and negatively charged (acidic) amino acids may include glutamic acid and aspartic acid; and among amino acids with an uncharged amino acid, nonpolar amino acids may include glycine, alanine, valine, leucine, isoleucine, methionine, phenylalanine, tryptophan, and proline, and polar or hydrophilic amino acids include serine, threonine, cysteine, tyrosine, asparagine and glutamine, and among the nonpolar amino acids, aromatic amino acids may include phenylalanine, tryptophan and tyrosine. In addition, the variant may include deletion or addition of amino acids having minimal effects on the properties and secondary structure of the polypeptide. For example, the polypeptide may be conjugated with a signal (or leader) sequence at the N-terminus of protein involved in protein transfer co-translationally or post-translationally. Furthermore, the polypeptide may be conjugated with another sequence or linker so as to identify, purify or synthesize the polypeptide.

In the present disclosure, "homology" refers to a percent of identity between two polynucleotide or polypeptide moieties. Homology between sequences from one moiety to another moiety may be determined by known corresponding techniques. For example, the homology may be determined by aligning sequence information and directly aligning sequence information between two polynucleotide molecules or two polypeptide molecules, for example, parameters such as scores, identity and similarity and the like using an easily available computer program. The computer program may be BLAST (NCBI), CLC Main Workbench (CLC bio), MegAlignTM (DNASTAR Inc) and the like. In addition, the homology between polynucleotides may be determined by confirming the size of degraded fragments by hybridizing polynucleotides under conditions that form a stable double strand between homologous regions, and then decomposing them with single-strand-specific nuclease.

In the present disclosure, 'homology' or 'identity' refers to the degree of similarity between two given amino acid sequences or base sequences, and may be expressed as a percentage. The terms homology and identity may be often used interchangeably.

The sequence homology or identity of a conserved polynucleotide or polypeptide may be determined by a standard alignment algorithm, and a default gap penalty established by the used program may be used together. Substantially, homologous or identical sequences may be generally hybridized with all or part of the sequences under mild or highly stringent conditions. It is obvious that hybridization also includes hybridization with a polynucleotide containing common codons or codons considering codon degeneracy.

Whether any two polynucleotide or polypeptide sequences have homology, similarity or identity, may be determined, for example, using a known computer algorithm such as "FASTA" program using a default parameter such as Pearson et al (1988) [Proc. Natl. Acad. Sci. USA 85]: 2444. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version), (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, using BLAST or ClustalW of National Center for Biotechnology Information database, homology, similarity, or identity may be determined.

The homology, similarity or identity of polynucleotides or polypeptides may be determined by comparing sequence information using for example, GAP computer program such as Needleman et al. (1970), J Mol Biol. 48:443, known in for example, Smith and Waterman, Adv. Appl. Math (1981) 2:482. In summary, GAP program may be defined as a value of dividing the total number of symbols in the shorter of two sequences by the number of similarly aligned symbols (i.e., nucleotides or amino acids). Default parameters for the GAP program may include (1) a binary comparison matrix (containing values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix), disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or gap opening penalty 10, gap extension penalty 0.5); and (3) no penalty for end gaps.

In the present disclosure, the term described in front of numerical values, "about" may be used to comprehensively mean numerical values in an equivalent or similar range to the numerical values described later, and in one embodiment, the equivalent or similar range may mean a range such as ±20%, ±15%, ±10%, ±5%, ±3%, ±2%, ±1% and the like of the described numerical values, but is not limited thereto.

In the present disclosure, even when only a numerical value is described, it may be interpreted as being used to comprehensively mean a numerical value in an equivalent or similar range to the described numerical value, and in one embodiment, the equivalent or similar range may mean a range such as ±20%, ±15%, ±10%, ±5%, ±3%, ±2%, ±1% and the like, but not limited thereto.

Hereinafter, the present disclosure will be described in more detail.

The present disclosure provides a microorganism expressing threonine export protein RhtC and a technology related to production of L-isoleucine using the same.

More specifically, the microorganism expressing threonine export protein RhtC
may comprise at least one selected from the group consisting of
(1) threonine export protein RhtC,
(2) a polynucleotide encoding the threonine export protein RhtC, and
(3) a vector comprising the polynucleotide.

The polynucleotide of the (2) and/or (3) may comprise
(a) a gene of the threonine export protein RhtC, or
(b) a gene of the threonine export protein RhtC and a promoter (for example, strong promoter) operably linked to the gene.

### Threonine export protein

The threonine export protein may be L-threonine export protein (L-threonine exporter, L-threonine efflux protein) RhtC. The L-threonine export protein RhtC is a protein mediating to export L-threonine out of cells, and is known as an inner membrane protein with five transmembrane domains. As analyzed by experimental topology, the C-terminus of the protein may be present in cytoplasm. In the present disclosure, the threonine export protein may be used interchangeably with RhtC protein or RhtC. The threonine export protein RhtC may be a wild type or a variant maintaining original functions and/or desired functions. The amino acid sequence of the threonine export protein may be obtained from known database such as GenBank of NCBI and the like.

The threonine export protein RhtC may be threonine export protein derived from *Escherichia coli (E. coli).*

In one specific embodiment, the threonine export protein RhtC may comprise an amino acid sequence having identity of 70% or more to an amino acid sequence of SEQ ID NO: 11, or an amino acid sequence having identity of 70% or more to an amino acid sequence in which the amino acid corresponding to the 62th position of SEQ ID NO: 11 is substituted with an amino acid different from the original. More specifically, the threonine export protein RhtC may comprise an amino acid sequence having identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more (the upper limit may be 100% or less than 100%) to an amino acid sequence of SEQ ID NO:11, or an amino acid sequence having identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more (the upper limit may be 100% or less than 100%) to an amino acid sequence of an amino acid sequence in which the amino acid corresponding to the 62th position of SEQ ID NO:11 is substituted with an amino acid different from the original. As the amino acid corresponding to the 62th position of SEQ ID NO: 11 is substituted with an amino acid different from the original, the activity of threonine export protein RhtC may be enhanced. In one embodiment, the amino acid corresponding to the 62th position of SEQ ID NO: 11 may be leucine (Leu, L). In one embodiment, the amino acid different from the original may be serine (Ser, S). In one embodiment, the amino acid sequence in which the amino acid corresponding to the 62th position of SEQ ID NO: 11 is substituted with serine may be expressed as SEQ ID NO: 13. As the amino acid corresponding to the 62th position of SEQ ID NO: 11 (leucine; Leu, L) is substituted with serine (ser, S), the activity of threonine export protein may be enhanced.

In one embodiment, the threonine export protein RhtC may be interpreted as including a protein having homology or identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more (the upper limit may be 100% or less than 100%) to a protein comprising the amino acid sequence of SEQ ID NO: 11 or the amino acid sequence in which the amino acid corresponding to the 62th position of SEQ ID NO: 11 is substituted with another amino acid different from the original, as long as the original functions (threonine export function) and/or desired functions (L-isoleucine production capacity (function of giving and/or increasing production capacity of L-isoleucine)) are maintained with the protein comprising the amino acid sequence of SEQ ID NO: 11 or an amino acid sequence in which the amino acid corresponding to the 62th position of SEQ ID NO: 11 is substituted with another amino acid different from the original.

### Polynucleotide encoding threonine export protein RhtC

In the present disclosure, a polynucleotide encoding threonine export protein RhtC may comprise a gene encoding the threonine export protein RhtC described above.

For the gene, due to codon degeneracy, or in consideration of preferred codons (codon optimization) in a living organism to express the protein, various modifications may be made in coding regions within a range without changing the amino acid sequence of the polypeptide. In one specific embodiment, the gene encoding threonine export protein RhtC of SEQ ID NO: 11 may comprise the nucleic acid sequence of SEQ ID NO: 12, and the gene encoding the protein comprising the amino acid sequence (for example, SEQ ID NO: 13) in which the amino acid corresponding to the 62th position of SEQ ID NO: 11 is substituted with serine may comprise the nucleic acid sequence of SEQ ID NO: 14, respectively. The nucleic acid sequence may comprise a mutated nucleic acid sequence within a range that does not change the encoded amino acid sequence, in consideration of codon degeneracy and/or codon optimization.

In one embodiment, the polynucleotide encoding the threonine export protein RhtC may further comprise a promoter, in addition to the gene encoding threonine export protein RhtC described above.

The promoter may be operably linked to the gene. More specifically, the polynucleotide encoding threonine export protein RhtC may comprise a promoter and a coding gene of the threonine export protein RhtC, in the direction from 5' to 3'.

In the present disclosure, the term "operably linked" may mean being functionally linked to the gene so that the promoter can perform transcriptional regulation of the gene. The operably linking may be performed using genetic recombination techniques known in the art, and for example, it may be performed by common site-specific DNA cleavage and linkage, but is not limited thereto.

The promoter may be a strong promoter which enhances expression (transcription) of an operably linked gene. In one specific embodiment, the promoter may comprise the nucleic acid sequence of SEQ ID NO: 15. In another embodiment, the promoter may be selected from known strong promoters, and for example, it may be at least one selected from the group consisting of cj1 to cj7 promoters (Korean Granted Patent No. 10-0620092), lac promoter, trp promoter, trc promoter, tac promoter, lambda phage PR promoter, PL promoter, tet promoter, gapA promoter, SPL1 promoter, SPL7 promoter, SPL13(sm3) promoter (Korean Granted Patent No. 10-1783170), O2 promoter (Korean Granted Patent No. 10-1632642), tkt promoter, yccA promoter, PlysC promoter (for example, PlysCP1 promoter (US 8426577 B2)) and the like, but not limited thereto.

### Vector comprising the polynucleotide

In the present disclosure, a vector comprising the polynucleotide may mean a DNA structure which comprises the afore-mentioned gene, or gene and promoter, and is capable of replication/expression (transcription). The vector may be expressed as a recombinant vector and an expression vector.

The vector is not particularly limited as long as it is capable of replication/transcription in a host cell, and may be selected among all commonly used vectors. In one embodiment, the vector may be present in a plasmid form in a host cell, or may be in a form of a vector which can be inserted into genome (chromosome) of a host cell, but not limited thereto.

The vector may mean a DNA structure comprising the polynucleotide operably linked to an appropriate regulatory sequence so as to express target protein (threonine export protein RhtC) in a suitable host cell. The regulatory sequence may comprise a promoter capable of initiating transcription, any operator sequence for regulating transcription, a sequence encoding an appropriate mRNA ribosome binding site, and/or a sequence which regulates termination of transcription and/or translation. The vector may be expressed independently of genome (chromosome) of the host cell, or integrated into genome of the host cell, after being transformed into a suitable host cell.

Examples of the commonly used vectors may include a plasmid, cosmid, virus, bacteriophage or the like in a natural state or a recombinant state. For example, as the vector, pWE15, M13, MBL3, MBL4, IXII, ASHII, APII, t10, t11, Charon4A, and Charon21A and the like, which are phage vectors or cosmid vectors, may be used, or pBR-based, pUC-based, pBluescriptII-based, pGEM-based, pTZ-based, pCL-based and pET-based and the like, which are plasmid vectors, may be used, but not limited thereto. In other embodiment, as the vector, pDC24, pDCM2, pDZ, pACYC177, pACYC184, pCL, pECCG117, pUC19, pBR322, pMW118, pCC1BAC vectors and the like may be exemplified, but not limited thereto.

In the present description, the available vector may be a known expression vector and/or a vector for insertion into host cell chromosome of a polynucleotide. The insertion into host cell chromosome of the polynucleotide may be accomplished by any method known in the art, for example, homologous recombination or CRISPR system, but not limited thereto.

The vector may further comprise a selection marker for confirming transformation of the host cell (for example, introduction to the host cell (for example, insertion into chromosome)). The selection marker is to select cells transformed with the vector, that is, to confirm introduction into a cell or insertion into chromosome of the polynucleotide, and may be used by being selected from genes giving selectable phenotypes such as drug resistance, auxotrophy, resistance to cytotoxic agents or expression of surface proteins. Since only cells expressing a selection marker are present or show different phenotypes under the environment a selective agent is treated, transformed cells can be selected.

### Enhancement of polypeptide

In one embodiment, the threonine export protein RhtC of the present disclosure may be enhanced.

In the present disclosure, the term, "enhancement" of the polypeptide activity refers to increasing the activity of the polypeptide compared to intrinsic activity. The enhancement may be interchangeably used with terms such as activation, up-regulation, overexpression, increase and the like. Herein, the activation, enhancement, up-regulation, overexpression, and increase may include all showing an activity that it does not originally have, or showing an increased activity compared to intrinsic activity or activity before modification. The "intrinsic activity" refers to activity of a specific polypeptide originally possessed by a parent strain or non-modified microorganism before changing traits, when traits may be changed by genetic mutation due to natural or artificial factors. This may be interchangeably used with "activity before modification". That the activity of the polypeptide is "enhanced", "up-regulated", "overexpressed" or "increased", compared to intrinsic activity, refers to being enhanced compared to the activity and/or concentration (expression level) of a specific polypeptide originally possessed by a parent strain before transformed or non-modified microorganism.

The enhancement may be achieved by introducing a foreign polypeptide, or through enhancement of activity and/or increase in concentration (expression level) of an intrinsic polypeptide. The enhancement of the activity of the polypeptide may be confirmed from the activity level, expression level of the corresponding polypeptide or an increase in the amount of products by the corresponding polypeptide activity.

For enhancement of the activity of the polypeptide, various methods well known in the art may be applied, and as long as the activity of a desired polypeptide can be enhanced compared to a microorganism before modification, it is not limited. Specifically, it may use genetic engineering and/or protein engineering well known to those skilled in the art, which are ordinary methods of molecular biology, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure
may be by
1) an increase of the copy number in a cell of a polynucleotide encoding the polypeptide;
2) replacement a gene expression regulatory region on chromosome encoding the polypeptide with a sequence with strong activity;
3) modification of a base sequence encoding an initiation codon or 5'-UTR region of gene transcriptome encoding the polypeptide;
4) modification of the amino acid sequence of the polypeptide to enhance the polypeptide activity;
5) modification of a polynucleotide sequence encoding the polypeptide to enhance the polypeptide activity (for example, modification of a polynucleotide sequence of the polypeptide gene to encode the polypeptide modified to enhance the activity of the polypeptide);
6) introduction of a foreign polypeptide showing the activity of the polypeptide or a foreign polynucleotide encoding the same;
7) codon optimization of a polynucleotide encoding the polypeptide;
8) transforming or chemically modifying the tertiary structure of the polypeptide by analyzing it to select an exposed site;
9) regulation of cellular localization of the protein (polypeptide); or
10) a combination of at least 2 selected from the 1) to 9), but not particularly limited thereto.

More specifically,
the 1) increase of the copy number in a cell of a polynucleotide encoding the polypeptide, may be achieved by introduction a vector capable of being replicated and functioning regardless of a host, in which a polynucleotide encoding the corresponding polypeptide is operably linked. Otherwise, the polynucleotide encoding the corresponding polypeptide may be achieved by introduction of 1 copy or 2 copies or more into chromosome in a host cell. The introduction into the chromosome may be performed by introducing a vector which can insert the polynucleotide into chromosome in a host cell into the host cell, but not limited thereto.

The 2) replacement a gene expression regulatory region (or expression regulatory sequence) on chromosome encoding the polypeptide with a sequence with strong activity, may be mutation occurrence on the sequence, or replacement with a sequence with stronger activity, by deletion, insertion, non-conservative or conservative substitution or a combination thereof to further enhance the activity of the expression regulatory region. The expression regulatory region, is not particularly limited thereto, but may comprise a promoter, an operator sequence, a sequence encoding a ribosome binding site, and a sequence regulating termination of transcription and translation and the like. As one example, it may be replacing the original promoter with a strong promoter, but not limited thereto.

The 3) modification of a base sequence encoding an initiation codon or 5'-UTR region of gene transcriptome encoding the polypeptide, may be for example, substituting with a base sequence encoding another initiation codon with a higher polypeptide expression rate compared to the intrinsic initiation codon, but not limited thereto.

The modification of the amino acid sequence or polynucleotide sequence of 4) and 5) may be mutation occurrence on the sequence by deletion, insertion, non-conservative or conservative substitution or a combination thereof for the amino acid sequence of the polypeptide or the polynucleotide sequence encoding the polypeptide to enhance the activity of the polypeptide, or replacement with an amino acid sequence or polynucleotide sequence improved to have stronger activity, but not limited thereto. The replacement may be performed specifically by inserting a polynucleotide into chromosome by homologous recombination, but not limited thereto. Then, the used vector may further comprise a selection marker for confirming insertion of chromosome.

The 6) introduction of a foreign polynucleotide showing the activity of the polypeptide may be introduction of a foreign polynucleotide encoding a polypeptide showing the same/similar activity to the polypeptide into a host cell. There is no limitation on the origin or sequence of the foreign polynucleotide as long as it shows the same/similar activity to the polypeptide. The method used for the introduction may be performed by appropriately selecting a known transformation method by those skilled in the art, and as the polypeptide is produced by expressing the introduced polynucleotide, the activity may be increased.

The 7) codon optimization of a polynucleotide encoding the polypeptide may be codon optimization to increase transcription or translation of the intrinsic polynucleotide into a host cell, or codon optimization thereof to achieve optimized transcription and translation of the foreign polynucleotide in a host cell.

The 8) transforming or chemically modifying the tertiary structure of the polypeptide by analyzing it to select an exposed site, may be for example, transforming or modifying by determining template protein candidates according to the sequence similarity level by comparing sequence information of a polypeptide to be analyzed with database in which sequence information of base proteins is stored, and confirming the structure based on this, and selecting an exposed site to be transformed or chemically modified.

The 9) regulation of cellular localization of the protein (polypeptide), may be targeting the protein (polypeptide) into a space in a specific organelle in a cell or in a specific cell. For example, it may be targeting into periplasm or cytoplasm through addition or removal of a leader sequence functioning to targeting of the protein (polypeptide), but not limited thereto.

Such enhancement of the polypeptide activity may be increasing the activity or concentration expression level of the corresponding polypeptide based on the activity or concentration of the polypeptide expressed in a wild type or microbial strain before modification, or increasing the amount of products produced from the corresponding polypeptide, but not limited thereto.

In one specific embodiment, the threonine export protein RhtC of the present disclosure may be enhanced by replacing a promoter of a gene encoding the protein with a strong promoter. The strong promoter may be CJ1 to CJ7 promoters (U.S. Granted Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Granted Patent US 10584338 B2), O2 promoter (U.S. Granted Patent US 10273491 B2), tkt promoter, yccA promoter, PlysC promoter (for example, PlysCP1 promoter (US 8426577 B2)), but not limited thereto.

### Microorganism expressing threonine export protein

As described above, the present disclosure provides a microorganism expressing threonine export protein RhtC.

In the present disclosure, "microorganism expressing threonine export protein RhtC" may mean a case where a microorganism which did not previously express threonine export protein RhtC has been made to express the threonine export protein RhtC, and/or a case where a microorganism which previously expressed threonine export protein RhtC has been made to overexpress the threonine export protein RhtC.

The microorganism is characterized by having L-isoleucine production capacity, or having increased L-isoleucine production capacity, compared to a homogeneous microorganism or wild type, which does not express the threonine export protein RhtC.

In one embodiment, the microorganism expressing the threonine export protein RhtC,
may comprise at least one selected from the group consisting of
(1) threonine export protein RhtC,
(2) a polynucleotide encoding the threonine export protein RhtC, and
(3) a vector comprising the polynucleotide.

Expressed differently, the microorganism expressing threonine export protein RhtC may be a recombinant microorganism in which a polynucleotide encoding the threonine export protein RhtC and/or a vector comprising the polynucleotide is introduced (transformed) into a host cell (also represented by a microorganism or parent strain).

When the microorganism (a wild type or microorganism in which the polynucleotide and/or vector encoding the threonine export protein RhtC is not introduced) as the host cell has L-isoleucine production capacity, the recombinant microorganism may have increased L-isoleucine production capacity, compared to a wild type and/or a host cell in which the polynucleotide and/or vector are not introduced. When the microorganism (a wild type or microorganism in which the polynucleotide and/or vector encoding the threonine export protein RhtC is not introduced) as the host cell does not have L-isoleucine production capacity, the recombinant microorganism may have given L-isoleucine production capacity.

In one embodiment, the microorganism producing L-isoleucine comprising at least one selected from the group consisting of threonine export protein RhtC, a polynucleotide encoding the threonine export protein RhtC, and a vector comprising the polynucleotide of the present disclosure, may have increased production capacity of L-isoleucine, compared to a microorganism not comprising at least one selected from the group consisting of the threonine export protein RhtC, a polynucleotide encoding the threonine export protein RhtC, and a vector comprising the polynucleotide. The microorganism not comprising at least one selected from the group consisting of the threonine export protein RhtC, a polynucleotide encoding the threonine export protein RhtC, and a vector comprising the polynucleotide may be a homogenous microorganism or wild-type microorganism which does not express threonine export protein RhtC.

In the present disclosure, "microorganism" encompasses single-celled bacteria, and may be interchangeably used with "cell". In the present disclosure, the microorganism before being mutated (transformed) to express the threonine export protein RhtC may be represented by "parent strain (or parent microorganism) or host cell", to distinguish from the mutated microorganism (recombinant microorganism).

In one embodiment, the microorganism, for example, microorganism as a host cell, may be (1) a microorganism naturally having L-isoleucine production capacity, (2) a microorganism naturally with no or significantly little L-isoleucine production capacity, and/or (3) a microorganism which becomes to have L-isoleucine production capacity or has improved L-isoleucine production capacity as a mutation is introduced (transformed) into the microorganism naturally having L-isoleucine production capacity and/or microorganism naturally with no or significantly little L-isoleucine production capacity. In one specific embodiment, the microorganism as a host cell may be *Corynebacterium glutamicum* KCCM12739P (CA10-3101, US 2023-0098971 A1) or *Corynebacterium glutamicum* KCCM11248P (Korean Granted Patent No. 10-1335789), but not limited thereto.

In one specific embodiment, the microorganism may be a microorganism of the genus *Corynebacterium.* The *Corynebacterium* sp. microorganism may include *Corynebacterium glutamicum, Corynebacterium ammoniagenes, Brevibacterium lactofermentum, Brevibacterium flavum, Corynebacterium thermoaminogenes, Corynebacterium efficiens,* and the like, but not limited thereto. Specifically, the *Corynebacterium* sp. microorganism may be *Corynebacterium glutamicum.*

In one specific embodiment, the microorganism may be a microorganism in which the biosynthesis pathway of L-isoleucine is additionally enhanced to increase the produced amount of L-isoleucine, but not limited thereto.

In one specific embodiment, the microorganism may be a microorganism in which inhibition of the feedback of L-threonine dehydratase is released.

In the present disclosure, the term, "threonine dehydratase (EC 4.3.1.19)" is an enzyme producing 2-ketobutyrate from threonine, and is known to be encoded by ilvA gene and undergo feedback inhibition by L-isoleucine. The *Corynebacterium* sp. microorganism synthesizes L-isoleucine using pyruvate and 2-ketobutyrate as precursors through 3 intermediate metabolites. The amino acid sequence of the threonine dehydratase may be obtained in known database, GenBank of NCBI or US 2023-0098971 A1, US 10982244 B2.

In one specific embodiment, in the microorganism, the feedback inhibition of homoserine dehydrogenase may be released.

In the present disclosure, the term, "homoserine dehydrogenase (EC:1.1.1.3)" is an enzyme which is encoded by hom gene and catalyzes synthesis of homoserine. Homoerine dehydrogenase is known to undergo feedback inhibition by isoleucine. The amino acid sequence of the homoserine dehydrogenase may be obtained from known database GenBank of NCBI or US 10982244 B2.

In one specific embodiment, in the microorganism, feedback inhibition of aspartate kinase may be released. In the present disclosure, the term, "aspartate kinase (EC: 2.7.2.4)" is encoded by lysC gene, and the amino acid sequence of the aspartate kinase may be obtained from known database, GenBank of NCBI or US 10662450 B2.

In the present disclosure, the term, the release of feedback inhibition may mean that the activity of a polypeptide, protein or enzyme may be increased or enhanced compared to the intrinsic activity or that the activity is not inhibited compared to the intrinsic activity. In one specific embodiment, the microorganism may be a microorganism in which a genetic mutation (R407H) is further introduced into hom gene (US 10982244 B2), and/or a genetic mutation (T381A, F383A and/or V323A) is further introduced into ilvA gene (US 2023-0098971 A1, US 10982244 B2), and/or a genetic mutation (L377K) (U.S. Granted Patent US 10662450 B2) is further introduced into lysC gene encoding aspartate kinase.

The introduction of the polynucleotide or vector may be performed by appropriately selecting a known transformation method by those skilled in the art. In the present disclosure, the term, "transformation" refers to introducing a polynucleotide encoding a target protein (threonine export protein RhtC) or a vector comprising the same into a host cell to make a protein encoding the polynucleotide to be expressed in the host cell. As long as the transformed polynucleotide can be expressed in a host cell, all of them may be comprised regardless of whether it is inserted and located in chromosome of the host cell and is located outside the chromosome. In addition, the polynucleotide may comprise DNA and/or RNA encoding a target protein. As long as the polynucleotide is introduced and expressed in a host cell, there is no limitation in the type to be introduced. For example, the polynucleotide may be introduced into a host cell in a form of an expression cassette, which is a genetic structure comprising all elements required for self-expression. The expression cassette may commonly comprise expression regulatory elements such as a promoter operably linked to the polynucleotide, a transcription termination signal, a ribosome binding site and/or a translation termination signal and the like. The promoter is as described above. The expression cassette may be a form of an expression vector capable of self-replication. In addition, the polynucleotide may be operably linked to a sequence required for expression in a host cell by being introduced into a host cell in its own form. In the above, the term "operably linked" may mean that expression regulatory elements (e.g., promoter) and a polynucleotide are functionally linked to perform transcription regulation (e.g., transcription initiation) of a polynucleotide encoding a target protein (threonine export protein RhtC). The operably linking may be performed using a gene recombination technology known in the art, and for example, it may be performed by common site-specific DNA cleavage and linkage, but not limited thereto.

The method for transforming the polynucleotide into a host cell may be performed by any method for introducing a nucleic acid into a cell (microorganism), and it may be performed by appropriately selecting a transformation technology known in the art depending on the host cell. As the known transformation method, electroporation, electeopulse, calcium phosphate (CaPO₄) precipitation, calcium chloride (CaCl₂) precipitation, microinjection, polyethylene glycol (PEG) precipitation (polyethylene glycol-mediated uptake), DEAE-dextran method, cation liposome method, lipofection, lithium acetate-DMSO method and the like may be exemplified, but not limited thereto.

Introduction (insertion) into host cell genome (chromosome) of the polynucleotide may be performed by appropriately selecting a known method by those skilled in the art. For example, it may be performed by common homologous recombination, or using an RNA-guided endonuclease system (or CRISPR system; for example, (a) RNA-guided endonuclease (e.g., Cas9 protein, etc.), an encoding gene thereof, or a vector comprising the gene; and (b) at least one selected from the group consisting of mixtures (for example, mixture of RNA-guided endonuclease protein and guide RNA, etc.), complexes (for example, ribonucleic acid fusion protein (RNP)), and recombinant vectors (for example, vector comprising an RNA-guided endonuclease encoding gene and guide RNA-encoding DNA together, etc.) comprising guide RNA (e.g., single guide RNA (sgRNA), etc.), encoding DNA thereof, or a vector comprising the DNA and the like), but not limited thereto.

In the present disclosure, the term "vector" means a DNA preparation containing a base sequence of a polynucleotide encoding the target protein operably linked to an appropriate regulatory sequence to express a target protein in a suitable host. The regulatory sequence may comprise a promoter capable of initiating transcription, any operator sequence for regulating transcription, a sequence encoding a suitable mRNA ribosome binding site, and/or a regulatory regulating termination of transcription and/or translation. The vector may be expressed regardless of genome (chromosome) of the host cell, or integrated in the genome of the host cell, after being transformed in a suitable host cell.

### Use of producing L-isoleucine

As described above, the microorganism expressing threonine export protein RhtC provided in the present disclosure is characterized by having L-isoleucine production capacity, or having increased L-isoleucine production capacity, compared to a homogeneous microorganism or wild type which does not express the threonine export protein RhtC.

The L-isoleucine production capacity may encompass not only a L-isoleucine producing function, but also an export/secretion function of L-isoleucine. The L-isoleucine production capacity may be confirmed by the isoleucine concentration, sugar consumption capacity and the like in a medium, but not limited thereto.

In one embodiment, the L-isoleucine production capacity of the microorganism expressing the threonine export protein RhtC may be increased by about 30% or more (i.e., about 1.3 times or more), about 40% or more (i.e., about 1.4 times or more), about 50% or more (i.e., about 1.5 times or more), about 60% or more (i.e., about 1.6 times or more), about 70% or more (i.e., about 1.7 times or more), about 80% or more (i.e., about 1.8 times or more), about 90% or more (i.e., about 1.9 times or more), about 100% or more (i.e., about 2 times or more), about 110% or more (i.e., about 2.1 times or more), about 120% or more (i.e., about 2.2 times or more), or about 130% or more (i.e., about 2.3 times or more) (the upper limit is not particularly limited, and for example, it may be about 300%, about 200%, or about 150%, but not limited thereto), compared to the L-isoleucine production capacity (for example, L-isoleucine concentration (g/L), sugar consumption capacity, L-isoleucine export capacity, etc. in a medium) (100%) of a parent strain (host cell, a homogeneous microorganism or wild-type microorganism which does not express threonine export protein RhtC), but not limited thereto.

Accordingly, one embodiment of the present disclosure provides a composition for producing L-isoleucine comprising at least one selected from the group consisting of the threonine export protein RhtC, a polynucleotide encoding the same, a vector comprising the polynucleotide, and a microorganism expressing threonine export protein RhtC.

The composition of the present disclosure may further comprise any appropriate excipient commonly used in a composition for producing amino acids, and this excipient may be for example, a preservative, a wetting agent, a dispersant, a suspending agent, a buffer, a stabilizer, or a tonicifying agent, but not limited thereto.

In the composition of the present disclosure, the variant, polynucleotide, vector, strain, medium and L-amino acid and the like are as described in the other aspect.

Other embodiment provides a method for producing L-isoleucine, comprising culturing a microorganism expressing the threonine export protein RhtC.

Other embodiment provides a method for preparing a microorganism producing L-isoleucine, and/or a method for giving and/or increasing L-isoleucine production capacity of a microorganism, comprising introducing a polynucleotide encoding the threonine export protein RhtC or a vector comprising the polynucleotide into a host cell (microorganism) to transform the host cell.

Other embodiment provides a use for producing L-isoleucine, preparing a microorganism producing L-isoleucine, and/or giving and/or increasing L-isoleucine production capacity of a microorganism, of at least one selected from the group consisting of the threonine export protein RhtC, a polynucleotide encoding the same, a vector comprising the polynucleotide, and a microorganism expressing the threonine export protein RhtC.

The threonine export protein RhtC, polynucleotide encoding the same, vector comprising the polynucleotide, microorganism expressing the threonine export protein RhtC, host cell, transformation and the like are as described above.

In the method for producing L-isoleucine comprising culturing a microorganism expressing the threonine export protein RhtC, the culturing may be performed in a suitable medium for culturing of a microorganism (microbial growth and/or L-isoleucine production, etc.). The medium is well known to those skilled in the art to which the present disclosure pertains. The method may further comprise recovering L-isoleucine from the cultured microorganism, medium or both of them, after the culturing.

In the method, the culturing a microorganism is not particularly limited thereto, but it may be performed by a known batch culture method, continuous culture method, fed-batch culture method, or the like. At this time, the culture conditions, are not particularly limited thereto, but appropriate pH (for example, pH 5 to 9, specifically, pH 6 to 8, most specifically, pH 7.2) may be adjusted using a basic compound (e.g.: sodium hydroxide, potassium hydroxide, or ammonia) or acidic compound (e.g.: phosphoric acid, sulfuric acid, phosphate, sulfate, etc.), and the aerobic condition may be maintained by introducing oxygen or an oxygen-containing gas mixture into the culture. The culture temperature may be maintained at 20 to 45°C, or 25 to 40°C, and it may be cultured for about 10 to 160 hours, but not limited thereto. L-isoleucine produced by the culturing may be secreted into the medium or remain within the cells.

The medium available for the culturing may use at least one individually or at least two in combination, which are selected from the group consisting of sugar and carbohydrates (e.g.: glucose, sucrose, lactose, fructose, maltose, molasse, starch and cellulose), oils and fats (e.g.: soybean oil, sunflower seed oil, peanut oil and coconut oil), fatty acids (e.g.: palmitic acid, stearic acid and linoleic acid), alcohols (e.g.: glycerol and ethanol), organic acids (e.g.: acetic acid) and the like, as a carbon source, but not limited thereto. As a nitrogen source, at least one individually or at least two in combination, which are selected from the group consisting of nitrogen-containing organic compounds (e.g.: peptone, yeast extract, broth, malt extract, corn steep liquor, soybean meal and urea), inorganic compounds (e.g.: ammonium sulfate, ammonium chloride, ammonium phosphate, ammonium carbonate, and ammonium nitrate) and the like, may be used, but it is not limited thereto. As a phosphorus source, at least one individually or at least two in combination, which are selected from the group consisting of potassium phosphate, potassium dihydrogen phosphate, dipotassium hydrogen phosphate, nitrogen-containing salts corresponding thereto and the like, may be used, but it is not limited thereto. In addition, the medium may comprise essential growth-promoting substances such as other metal salts (e.g.: magnesium sulfate, iron sulfate, manganese sulfate, etc.), amino acids, and/or vitamins and the like.

Recovering L-isoleucine may involve collecting desired amino acids from a medium, cultured solution, or microorganism using an appropriate method known in the art according to the culture method. For example, the recovering may be performed by at least one method selected from centrifugation, filtration, anion exchange chromatography, crystallization, HPLC and the like. The method for recovering L-isoleucine may additionally comprise a purification step before, at the same time as, or after then.

Other embodiment provides a composition, method, product, process or use characterized by at least one element disclosed in the present disclosure.

### [ADVANTAGEOUS EFFECTS]

The microorganism expressing threonine export protein RhtC provided in the present disclosure has an improved L-isoleucine production capacity compared to a microorganism not expressing the same, and thus, it can effectively produce L-isoleucine using the same. Accordingly, widespread industrial utilization such as food, feed and medicine and the like, which utilize L-isoleucine of the microorganism can be expected.

### [MODE FOR INVENTION]

Hereinafter, the present invention will be described in more detail by examples, but they are illustrative only, but they are not intended to limit the scope of the present invention. It is obvious to those skilled in the art that the examples described below can be modified without departing from the essential gist of the invention.

### Example 1. Construction of a recombinant vector for introducing a threonine export protein RhtC

### Example 1-1: Construction of a plasmid for gene insertion

In order to insert rhtC gene encoding threonine export protein RhtC, NCg12533 of host cell (*Corynebacterium glutamicum* ATCC13032) genome was used as an insertion site. To substitute the NCg12533 gene with foreign rhtC, a vector for NCg12533 deletion and target gene insertion was constructed. For producing the vector, using chromosome of *Corynebacterium glutamicum* ATCC13032 as a template, and using the primer pairs of SEQ ID NO: 1 and SEQ ID NO: 2, and SEQ ID NO: 3 and SEQ ID NO: 4, PCR was performed, respectively. The primer sequences used for performing each PCR were as shown in Table 1 below.

**[Table 1]**

| SEQ ID NO: | Name | Sequence |
|---|---|---|
| 1 | Primer1 | TGAATTCGAGCTCGGTACCC TCATATTGAATTTCGCCCCA |
| 2 | Primer2 | CCTAAGCAGGTTGATTTagt actCCGCTGGAAAACATTTT |
| 3 | Primer3 | AAAATGTTTTCCAGCGGagt actAAATCAACCTGCTTAGG |
| 4 | Primer4 | GTCGACTCTAGAGGATCCCC TCCCACCAAATCTGCGCCCA |

PfuUltraTM high-fidelity DNA polymerase (Stratagene) was used as polymerase for PCR reactions, and PCR conditions are denaturation at 95°C for 30 seconds; annealing at 55°C for 30 seconds; and polymerization at 72°C for 1 minute, and the denaturation, annealing and polymerization of these conditions were repeated 28 times. As a result, DNA fragments of 1020bp and 1043bp, respectively, were obtained. The obtained DNA products were purified using PCR Purification kit (QUIAGEN), and the purified amplified products were cloned using pDC24 vector (SEQ ID NO: 16) treated with restriction enzyme smal, and then heat-treated at 65°C for 20 minutes and Infusion Cloning Kit (TaKaRa) according to the provided manual, to construct the vector for NCg11756 deletion and target gene insertion, pDC24△N2533 was constructed.

### Example 1-2: Replacement of a promoter and construction of a plasmid for mutation instruction

To intensify the activity of rhtC, a gene (SEQ ID NO: 12) encoding RhtC (wild type; SEQ ID NO: 11) of *E*. *coli* W3110 (ATCC 27325), and a gene (SEQ ID NO: 14) encoding a mutant RhtC (L62S; SEQ ID NO: 13) derived from the wild type were introduced (US 20230012923 A1). By replacing promoters of rhtC (wild type) and rhtC(L62S) genes with PlysCP1(SEQ ID NO: 15), using PlysCP1 promoter (US 8426577 B2) of which some sequences were changed based on PlysC, plasmids for enhanching the activity of RhtC were constructed.

The sequences of used protein and DNAwere as the following Table 2:

**[Table 2]**

| SEQ ID NO: | Description | Amino acid sequence (N→C) or DNA sequence (5'→3') |
|---|---|---|
| 11 | rhtC wild-type | MLMLFLTVAMVHIVALMSPGPDFFFVSQTAVSRSRKEAM MGVLGITCGVMVWAGIALLGLHLIIEKMAWLHTLIMVGGGL YLCWMGYQMLRGALKKEAVSAPAPQVELAKSGRSFLKGL LTNLANPKAIIYFGSVFSLFVGDNVGTTARWGIFALIIVETLA WFTVVASLFALPQMRRGYQRLAKWIDGFAGALFAGFGIHL IISR |
| 12 | rhtC wild-type coding DNA | atgttgatgttatttctcaccgtcgccatggtgcacattgtggcgcttatgagccccgg tcccgatttcttttttgtctctcagaccgctgtcagtcgttcccgtaaagaagcgatgat gggcgtgctgggcattacctgcggcgtaatggtttgggctgggattgcgctgcttgg cctgcatttgattatcgaaaaaatggcctggctgcatacgctgattatggtgggcgg tggcctgtatctctgctggatgggttaccagatgctacgtggtgcactgaaaaaag aggcggtttctgcacctgcgccacaggtcgagctggcgaaaagtgggcgcagttt cctgaaaggtttactgaccaatctcgctaatccgaaagcgattatctactttggctcg gtgttctcattgtttgtcggtgataacgttggcactaccgcgcgctggggcatttttgc gctgatcattgtcgaaacgctggcgtggtttaccgtcgttgccagcctgtttgccctg ccgcaaatgcgccgtggttatcaacgtctggcgaagtggattgatggttttgccgg ggcgttatttgccggatttggcattcatttgattatttcgcggtga |
| 13 | rhtC L62S | MLMLFLTVAMVHIVALMSPGPDFFFVSQTAVSRSRKEAM MGVLGITCGVMVWAGIALLGLHSIIEKMAWLHTLIMVGGGL YLCWMGYQMLRGALKKEAVSAPAPQVELAKSGRSFLKGL LTNLANPKAIIYFGSVFSLFVGDNVGTTARWGIFALIIVETLA WFTVVASLFALPQMRRGYQRLAKWIDGFAGALFAGFGIHL IISR |
| 14 | rhtC L62S coding DNA | atgttgatgttatttctcaccgtcgccatggtgcacattgtggcgcttatgagccccgg tcccgatttcttttttgtctctcagaccgctgtcagtcgttcccgtaaagaagcgatgat gggcgtgctgggcattacctgcggcgtaatggtttgggctgggattgcgctgcttgg cctgcattcgattatcgaaaaaatggcctggctgcatacgctgattatggtgggcg gtggcctgtatctctgctggatgggttaccagatgctacgtggtgcactgaaaaaa gaggcggtttctgcacctgcgccacaggtcgagctggcgaaaagtgggcgcagt ttcctgaaaggtttactgaccaatctcgctaatccgaaagcgattatctactttggctc ggtgttctcattgtttgtcggtgataacgttggcactaccgcgcgctggggcatttttg cgctgatcattgtcgaaacgctggcgtggtttaccgtcgttgccagcctgtttgccct gccgcaaatgcgccgtggttatcaacgtctggcgaagtggattgatggttttgccg gggcgttatttgccggatttggcattcatttgattatttcgcggtga |
| 15 | PlysCP1 | ccatcttttggggtgcggagcgcgatccggtgtctgaccacggtgccccatgcgat tgttaatgccgatgctagggcgaaaagcacggcgagcagattgctttgcacttgat tcagggtagttgactaaagagttgctcgcgaagtagcacctgtcacttttgtctcaa atattaaatcgaatatcaatatatggtctgtttattggaacgcgtcccagtggctgag acgcatccgctaaagccccaggaaccctgtgcagaaagaaaacactcctctgg ctaggtagacacagtttattgtggtagagttgagcgggtaactgtcagcacgtaga tcgaaaggtgcacaaaggtggccctggtcgtacagaaatatggcggttcctcgct tgagagtgcggaacgcattagaaacgtcgctgaacggatcgttgccaccaaga aggctggaaatgatgtcgtggttgtctgctccgcaatgggagacaccacggatga acttctagaacttgcagcggcagtgaatcccgttccgccagctcgtgaaatggata |
| | | tgctcctgactgctggtgagcgtatttctaacgctctcgtcgccatggctattgag |

Specifically, in order to produce strains having the PlysCP1 promoter in which rhtC (Wild type) and rhtC(L62S) were introduced, PlysCP1 promoter fragments (WO2009/096689) were obtained using pDZ_lysCP1 as a template and using SEQ ID NO: 5 and SEQ ID NO: 6. PCR was performed using chromosome of E. coli W3110 as a template and using the primers of SEQ ID NO: 7 and SEQ ID NO: 10, and rhtC (wild type) fragments were obtained. In addition, PCR was performed using chromosome of E. coli W3110 as a template and using the primers of SEQ ID NO: 7 and SEQ ID NO: 8, and SEQ ID NO: 9 and SEQ ID NO: 10, and rhtC(L62S) fragments of which the 62th amino acid was substituted with serine were obtained. The primer sequences used to perform each PCR were as shown in Table 3 below.

**[Table 3]**

| SE Q ID NO: | Name | Sequence |
|---|---|---|
| 5 | Primer5 | AAAATGTTTTCCAGCGGAGTATTCAGGGTAGTTGACTAAA |
| 6 | Primer6 | AATAACATCAACATCTTTGTGCACCTTTCGATCT |
| 7 | Primer7 | AGATCGAAAGGTGCACAAAGATGTTGATGTTATTTCTCAC |
| 8 | Primer8 | ATTTTTTCGATAATCGAATGCAGGCCAAGC |
| 9 | Primer9 | GCTTGGCCTGCATTCGATTATCGAAAAAAT |
| 10 | Primer10 | CCTAAGCAGGTTGATTTAGTTCACCGCGAAATAATCAAAT |

PfuUltraTM high-fidelity DNA polymerase (Stratagene) was used as polymerase for PCR reactions, and PCR conditions are denaturation at 95°C for 30 seconds; annealing at 55°C for 30 seconds; and polymerization at 72°C for 1 minute, and the denaturation, annealing and polymerization of these conditions were repeated 28 times. As a result, 243 bp DNA fragments of the PlysCP1 promoter site, 661 bp DNA fragments of rhtC site, 220 bp and 471 bp DBA fragments of rhtC(L62S) site were obtained, respectively.

The amplified products were purified using PCR Purification kit (QUIAGEN), and used as inserted DNA fragments for vector construction. After treating the purified amplified products with restriction enzyme Scal, the pDC24△N2533 vector, heat-treated at 65°C for 20 minutes, and the amplified products, DNA fragments to be inserted, were mixed at a molar ration (M) of 1:2. Cloning was then performed using Infusion Cloning Kit (TaKaRa) according to the provided manual, to construct vectors for introducing the rhtC (wild type) and rhtC(L62S) mutant into chromosome, pDC24△N2533::PlysCP1_rhtC (Ec), and pDC24△N2533::PlysCP1_rhtC (Ec, L62S).

### Example 2: Production and evaluation of rhtC introduced strain in Corynebacterium glutamicum L-isoleucine producing strain (1)

Strains in which foreign rhtC (wild type) and rhtC(L62S) were introduced into genome through homologous recombination were produced, by transforming the vectors produced in Example 1.2 into an isoleucine producing strain, Corynebacterium glutamicum KCCM12739P (CA10-3101, Korean Granted Patent 10-2363913) by electroporation. The strains produced like this were named CA10-3136 (rhtC (wild type) introduced), and CA10-3135 (rhtC(L62S) introduced), respectively.

In order to confirm the increase and decrease effects of isoleucine productivity of the strains, they were cultured by the following method. The parent strain and the mutant strains were inoculated into a 250mL corner-baffled flask containing an isoleucine production medium of 25 mL, and then cultured with shaking at 200 rpm at 32°C for 60 hours, to produce L-isoleucine. The composition of the medium used in the present example is as follows.

### <Production medium>

(pH 7.2) glucose 10%(w/v), yeast extract 0.2%(w/v), ammonium sulfate 1.6%(w/v), potassium phosphate monobasic 0.1%(w/v), magnesium sulfate heptahydrate 0.1%(w/v), iron sulfate heptahydrate 10mg/L, manganese sulfate monohydrate 10 mg/L, biotin 200 µg/L

After completing culturing, using high-performanceliquid chromatography (HPLC), the produced amount of L-isoleucine (isoleucine content in the medium) was measured, and the concentration was shown in Table 4 below.

**[Table 4]**

| | Isoleucine concentration (g/L) | Increase in yield compared to parent strain (Δ,%p) |
|---|---|---|
| CA10-3101 (parent strain) | 2.1 | - |
| CA10-3136 | 3.7 | 1.6, 76% |
| [CA10-3101ΔN2533::PlysCP1_rhtC(Ec)] | | |
| CA10-3135[CA10-3101ΔN2533::PlysCP1_rhtC(Ec, L62S)] | 4.5 | 2.4, 114% |

As a result, as shown in Table 4 above, it was confirmed that the parent strain, *Corynebacterium glutamicum* CA10-3101 produced isoleucine of about 2.1 g/L. It was confirmed that the rhtC-introduced strain, CA10-3136 produced isoleucine of about 3.7 g/L and the productivity was increased by about 80% compared to the parent strain, and the rhtC(L62S)-introduced strain, CA10-3135 had an increased sugar consumption rate, and produced isoleucine of about 4.5 g/L, and therefore, the productivity compared to the parent strain increased by about 114%.

### Example 3: Production and evaluation of rhtC introduced strain in Corynebacterium glutamicum L-isoleucine producing strain (2)

The vector produced in Example 1.2 above was introduced into an L-isoleucine producing strain treated with NTG (N-Methyl-N'-nitro-N-nitrosoguanidine), KCJI-38 (KCCM11248P, Korean Granted Patent No. 10-1335789) strain, by the electric pulse method, and then was transformed by smearing on a selective medium containing kanamycin 25mg/L, to obtain mutant strains in which rhtC or rhtC(L62S) was introduced on the chromosome. The strains produced like this were named CA10-3137, CA10-3138, respectively. After that, by the same methods as Example 2, the L-isoleucine concentration in the cultured solution was measured, and the result was shown in Table 5 below.

**[Table 5]**

| | Isoleucine | Increase in yield |
|---|---|---|
| | concentration (g/L) | compared to parent strain (Δ,%p) |
| KCCM11248P (Parent strain) | 1.0 | |
| CA10-3137 | 1.8 | 0.8, 80% |
| [KCCM11248PΔN2533::PlysCP1 _rhtC(Ec)] | | |
| CA10-3138[KCCM11248PΔN2533::Plys CP1_rhtC(Ec, L62S)] | 2.4 | 1.4, 140% |

As a result, as shown in Table 5 above, it was confirmed that the parent strain, *Corynebacterium glutamicum* KCCM11248P produced isoleucine of about 1.0 g/L. It was confirmed that the rhtC-introduced strain, CA10-3137 had an increased sugar consumption rate, and produced isoleucine of about 1.8 g/L, and the productivity compared to the parent strain increased by about 80%, and it was confirmed that the rhtC(L62S)-introduced strain, CA10-3138 had an increased sugar consumption rate, and produced isoleucine of about 2.4 g/L, and the productivity compared to the parent strain increased by about 140%.

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

## Claims

1. A microorganism producing L-isoleucine, which expresses threonine export protein RhtC.

2. The microorganism producing L-isoleucine according to claim 1, wherein the microorganism comprises at least one selected from the group consisting of threonine export protein RhtC, a polynucleotide encoding the threonine export protein RhtC, and a vector comprising the polynucleotide.

3. The microorganism according to claim 1, wherein the threonine export protein RhtC comprises an amino acid sequence having identity of 70% or more with an amino sequence of SEQ ID NO: 11, or an amino acid sequence having identity of 70% or more with an amino acid sequence in which the amino acid corresponding to the 62th position of SEQ ID NO: 11 is substituted with an amino acid different from the original.

4. The microorganism according to claim 3, wherein the amino acid different from the original is serine.

5. The microorganism according to claim 1, wherein the threonine export protein RhtC is enhanced.

6. The microorganism according to claim 1, wherein the microorganism is a *Corynebacterium* sp. microorganism.

7. The microorganism according to claim 6, wherein the *Corynebacterium* sp. microorganism is *Corynebacterium glutamicum.*

8. The microorganism according to claim 1, wherein the microorganism has an increased production capacity of L-isoleucine, compared to a microorganism not comprising at least one selected from the group consisting of the threonine export protein RhtC, a polynucleotide encoding the threonine export protein RhtC, and a vector comprising the polynucleotide.

9. The microorganism according to claim 1, wherein in the microorganism, feedback inhibition of L-threonine dehydratase is released.

10. The microorganism according to claim 1, wherein in the microorganism, feedback inhibition of homoserine dehydrogenase is released.

11. A method for producing L-isoleucine, comprising culturing the microorganism according to any one claim of claim 1 to claim 10 in a medium.

12. The method for producing L-isoleucine according to claim 11, further comprising recovering L-isoleucine from the cultured microorganism, medium, or both of them.

13. A method for increasing an L-isoleucine production capacity of a microorganism, comprising introducing a polynucleotide encoding threonine export protein RhtC or a vector comprising the polynucleotide into a host cell to transform the host cell.

14. A use for producing L-isoleucine, of the microorganism according to any one claim of claim 1 to claim 10.

15. A composition, method, product, process, or use **characterized by** at least one element disclosed in the present disclosure.
